(19) Europäisches Patentamt
European Patent Office
Office européen des brevets

(11) Publication number: 0 237 271 A2

(12) # EUROPEAN PATENT APPLICATION

(21) Application number: 87301912.9

(22) Date of filing: 05.03.87

(51) Int. Cl.³: C 07 D 493/04
A 01 N 43/90

(30) Priority: 07.03.86 JP 48458/86

(43) Date of publication of application:
16.09.87 Bulletin 87/38

(84) Designated Contracting States:
CH DE FR GB LI

(71) Applicant: Nippon Kayaku Kabushiki Kaisha
11-2, Fujimi-cho 1 chome Chiyoda-ku
Tokyo(JP)

(84) Designated Contracting States:

(71) Applicant: KYOWA HAKKO KOGYO CO., LTD.
6-1, Ohte-Machi 1-chome
Chiyoda-ku Tokyo-to(JP)

(84) Designated Contracting States:

(72) Inventor: Taniguchi, Eiji
3-16-23 Mizutani Higashi-ku
Fukuoka-shi Fukuoka-ken(JP)

(72) Inventor: Masui, Akio
1-14 Minami-Nakano
Omiya-shi Saitama-ken(JP)

(74) Representative: Myerscough, Philip Boyd et al,
J.A.Kemp & Co. 14, South Square Gray's Inn
London, WC1R 5EU(GB)

(54) A sesquilignan derivative.

(57) Sesquilignan derivatives of the formula:

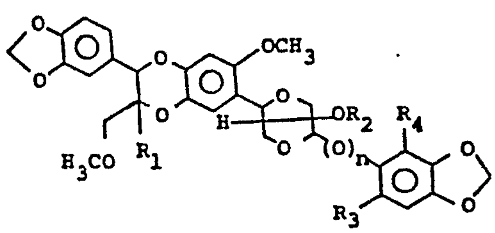

wherein $R_1$ is hydrogen or hydroxy, $R_2$ is hydrogen or acetyl, $R_3$ is hydrogen or methoxy, $R_4$ is hydrogen or methoxy and n is 0 or 1, with the priviso that when $R_1$ and $R_2$ are each hydrogen, $R_3$ is methoxy and $R_4$ is hydrogen, are pesticidally active.

EP 0 237 271 A2

# "A SESQUILIGNAN DERIVATIVE"

The present invention relates to a novel sesquilignan derivative, a method for extracting and isolating it from lopseed (Phryma leptostachya L.) and a pesticidal composition containing it.

Lopseed has long been known to kill house flies, but the active ingredients have not been fully elucidated. It was recently reported that an active ingredient was the sesquilignan derivative of the formula:

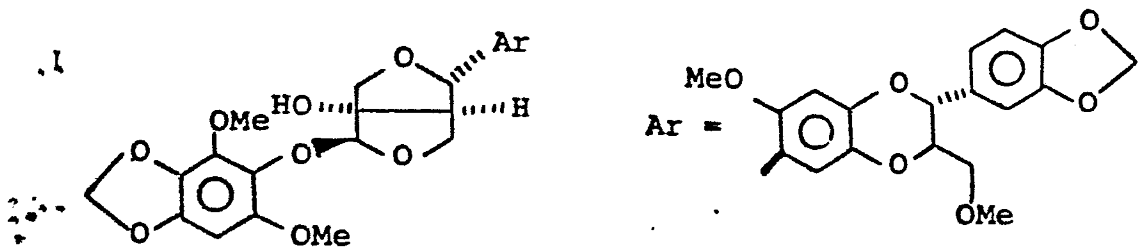

(Haedoxane A)

("The 21st Kagaku Kanren Shibu Godo Kyushu Taikai, Koen Yokoshu, July 13, 1984").

We have found that sesquilignan derivatives having an excellent insecticidal activity can be obtained by application of the extraction and/or isolation, as described in (A) and (B) below

respectively, from lopseed (Phryma leptostachya L.):

(A) A method for obtaining a crude extract containing a mixture of sesquilignan derivative of the formula:

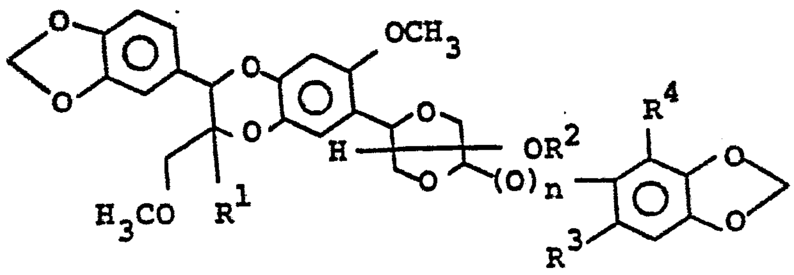

(I)

wherein $R^1$ is hydrogen or hydroxy, $R^2$ is hydrogen or acetyl, $R^3$ is hydrogen or methoxy, $R^4$ is hydrogen or methoxy and n is integer of 0 or 1, which comprises:

(1) extracting lopseed (Phryma leptostachya L.) with an organic solvent and removing the solvent from the extract solution to concentrate,

(2) removing the materials which are soluble in an acidic, alkaline or neutral aqueous solution by partition of concentrate obtained by (1) between an adequate organic solvent and the above aqueous solution, and concentrating the organic solution by removing the adequate organic solvent, and

(3) removing the non-polar-solvent-soluble materials (a) by partition of the concentrate of (2) between a non-polar solvent and a polar solvent and concentrating the polar solvent-solution, to obtain the crude extract or (b)

by addition of the organic solution mentioned above (2) into large volume of a non-polar solvent with stirring and decanting the non-polar solvent to obtain the curde extract.

(B) A method for separating a sesquilignan derivative of the formula (I) by applying chromatography technique to the crude extract of (3) in (A).

The detail methods of (A) and (B) are as follows.

At first, whole grass, preferably root of lopseed (Phryma leptostachya L.), either raw or dried, is extracted with organic solvent of preferably 1 to 10 times as much as the weight of the raw grass, for example, lower alcohols such as methanol and ethanol, ketones such as acetone and methyl ethyl ketone, esters such as ethyl acetate and butyl acetate, ethers such as diethyl ether and isopropyl ether, aromatic hydrocarbons such as benzene, toluene and xylene, halogenated hydrocarbons such as chloroform, methylene chloride and perchloroethylene or mixed solvents thereof, according to the conventional extracting method of plant components such as extraction with dipping or with shaking, or continuous extraction using soxhlet extractor.

After that, the organic solvent mentioned above is distilled off from the extract solution under an ordinary or reduced pressure to obtain a concentrate of adequate

volume (preferably less than one-tenth of the original volume). Then, the obtained concentrate of adequate volume was subjected to partition between ① acidic, alkaline or neutral solution and ② adequate organic solution to remove the materials which are soluble with the aqueous solution of ①.

As a preferable aqueous solution, there can be mentioned 0.5 to 2% aqueous solution of mineral acids such as hydrochloric acid, or an aqueous solution of alkali, such as 2 to 10% aqueous solution of sodium hydrogen carbonate or 0.5 to 2% aqueous solution of sodium hydroxide, or water.

As an adequate solvent, there can be mentioned ethyl acetate, butyl acetate, diethyl ether, isopropyl ether, benzene, toluene, xylene, p-cymene, chloroform, methylene chloride and perchloroethylene. Then the organic solvent-solution is concentrated to an adequate volume (preferably less than one-tenth of the original volume).

The concentrate obtained above is subjected to partition between a non-polar solvent and a polar solvent. Examples of the non-polar solvent include petroleum ether or aliphatic hydrocarbons ($C_{5-12}$) such as n-heptane, n-hexane, n-octane and n-undecane, and those of the polar solvent include methanol (containing preferably 0 to 20% v/v of water), ethanol (containing preferably 5 to 20% v/v of water), acetonitrile

(containing preferably 0 to 20% v/v of water) and methylcellosolve (containing preferably 0 to 20% v/v of water), among which are preferably used methanol and acetonitrile. The amount of the non-polar and polar solvents used is not particularly limited but 5 to 100 times and 5 to 150 times weight, respectively, as much as the concentrate are preferable.

Further, the polar solvent-solution is separated from the phase of non-polar solvent. The solvent of the polar solvent solution is distilled off by an ordinary or reduced pressure to obtain the objective crude extract as a resinous or viscous oil.

In addition, a method for removal of the substance soluble in non-polar solvent by partition between polar and non-polar solvents may be simplified by the procedure mentioned below. Namely, while a concentrated adequate solution is added into a large quantity of a non-polar solvent under stirring, the solvent, dissolving the objective compound, comes into the solution of the non-polar solvent, and so the objective compound as such is precipitated.

The crude extract thus obtained can be separated, for example, by means of the following adsorption chromatography. A column chromatography, semi-preparative thin layer chromatography, or high-speed liquid chromatography, using silica gel, alumina or Florisil as an adsorbent are

appropriate to the occasion.

As for the separation process of this invention, these chromatographies may be utilized either alone or in arbitrary combination of them. The solvent of lower polarity such as hexane, benzene or dichloromethane is preferably mixed with 5 to 95% of the solvent of higher polarity, for example, lower alcohols such as methanol and ethanol, lower ketones such as acetone and methyl ethyl ketone, acetonitrile, esters such as ethyl acetate and butyl acetate, or ethers such as diethyl ether and isopropyl ether is used as an eluent.

A column for high-speed liquid chromatography may be usually the one marketed generally, for example, Lichroprep-Si® 60 (Merck Corp.), μ-Porosil® (Waters Corp.), Resolve Si® (Waters Corp.) etc. Among the above-mentioned chromatographies for separation, an adsorption chromatography is preferable, by which a mixed solvent such as hexane/ethyl acetate or diethyl ether, or benzene/ethyl acetate or isopropyl ether is preferably used.

In addition, the crude extract can be separated also by means of partition chromatography. Namely, the fixed phase which is made of carriers such as silica gel treated by octadecylsilanol, octasilanol or phenylalkylsilanol is used. An aqueous solution containing preferably 5 to 95% of acetonitrile, methanol, ethanol, tetrahydrofuran,

dioxane etc. is used as an eluent. As a high-speed liquid chromatography with such a column for partition there may be used μ-Bondapak-$C_{18}$® (Waters Corp.), Novapak-$C_{18}$® (Waters Corp.), Radialpak-$C_8$ (Waters Corp.) or Lichrosorb RP-18® (Merck Corp.), and as a thin-layer chromatography plate, the marketed HPTLC plate RP-18 (Merck Corp.) may be also employed.

A combination of these adsorption and partition chromatographies has brought efficiently a fraction containing much novel sesquilignan derivative.

The derivative of the present invention may be used alone according to the purpose of application in their practical use, but they are generally formulated by blending suitable adjuvants to improve or stabilize the effects thereof and used as such or after diluted if necessary. The compounds of the invention can be formulated in the conventional manners well-known in the art in any convenient form such as dust, granule, microgranule, wettable powder, flowable powder, emulsion, microcapsule, oil, aerosol, heating fumigant (e.g. mosquito repellent of an incense type or electric type), fuming agents such as fogging, non-heating fumigant, or toxic feed.

Examples of said adjuvants are carrier (i.e. diluent) and other adjuvants such as spreader, emulsifying agent, wetting agent, dispersing agent, fixing agent or

disintegrator. Examples of the liquid carrier are aromatic hydrocarbons such as toluene or xylene; alcohols such as methanol, butanol or glycol; ketones such as acetone; amides such as dimethylformamide; sulfoxides such as dimethyl sulfoxide; methylnaphthalene; cyclohexane; animal or vegetable oils; fatty acids and esters thereof or the like as well as petroleum fractions such as keronsine or gas oil.

Examples of the solid carrier are clay, kaolin, talc, diatomaceous earth, silica, calcium carbonate, montmorillonite, bentonite, feldspar, quartz, alumina or saw dust.

Surfactants are generally used as an emulsifying or dispersing agent. Examples of them are anionic, cationic, non-ionic and amphoteric surfactants such as sodium salt of higher alcohol sulfate, stearyltrimethylammonium chloride, polyoxyethylene alkylphenyl ether or laurylbetaine.

Examples of the spreaders are polyoxyethylene nonylphenyl ether and polyoxyethylene lauryl ether. Examples of the wetting agents are polyoxyethylene nonylphenyl ether and dialkyl sulfosuccinates. Examples of the fixing agents are carboxymethylcellulose and polyvinyl alcohol. Examples of the disintegrators are sodium ligninsulfonate and sodium laurylsulfate.

Furthermore, it is possible to blend two or more compounds of the present invention to obtain an improved

insecticidal, nematicidal and acaricidal activities. In addition, it may be also possible to use a compound of the present invention simultaneously with other physiologically active substances.

It is further possible to increase the effect of the composition several times as much by adding synergists for pyrethroids such as piperonyl butoxide, sulfoxide or safroxane.

Although the compounds of the present invention are stable to light, heat, and oxidation or the like, antioxidant or ultraviolet absorbers such as phenols; e.g., BHT or BHA; arylamines, e.g., α-naphthyl amine; or benzophenone compound may be added as a stabilizer to prepare a composition which exhibits a higher stability, if necessary.

The content of active ingredients in the composition of the present invention varies depending on the conditions of use such as formulation form or application method, and is usually from 0.2 to 95% by weight, preferably from 0.5 to 80% by weight, although the active ingredients may be used alone in a special case.

The pesticidal (insecticidal, acaricidal or nematicidal composition of the present invention may be used in an amount which depends on the conditions such as formulation form, method or season for application. It is usually used in an amount of 0.5 to 100g/10a ($a = 100m^2$), preferably 1 to 50g/10a in terms of the active ingredient

for the agricultural and horticultural pesticide and the insecticide for forestry and pasturage, and in an amount of 2 to 200 $mg/m^2$, preferably 5 to 100 $mg/m^2$ in terms of the active ingredient for the hygienic insecticide. For example, 5 to 50g/10a of the active ingredient is used in the case of dust, 5 to 100g/10a thereof is used in the case of granule, and 1 to 50g/10a thereof is used in the case of emulsion. However, it may be possible, or even necessary, to use the active ingredient in an amount which is outside the range as specified above, in a special case.

The insect pests on which the insecticides, acaricides and nematicides of the present invention are effective are as follows: Hemiptera such as Nephotettix cincticeps, Sogatella furcifera, Niraparvata lugens, Laodelphax striatellus, Riptortus clavatus, Nezara viridula, Stephanis nashi, Trialeurodes vaporaviorum, Aphis gossypii, Myzus persicae and Vnaspis yanonensis; Lepidoptera such as Phyllonorycter ringoneella, Plutella xylostella, Promalactis inonisema, Adoxophyes orana, Leguminivora glycinivorella, Cnaphalocrocis medinalis, Chilo suppressalis, Ostrinia furnacalis, Mamestra brassicae, Pseudaletia separata, Spodoptera litura, Parnara guttata and Pieris rapae crucivora; Coleoptera such as Anomala cuprea, Popillia japonica, Echinocnemus soqameus, Lissorhoptrus oryzophilus, Anthonomus grandis, Oulema oryzae, Diabrotica undecimpunctata,

Leptinotarsa decemlineata, Anthrenus verbasci, Tenebroides mauritanicus, Sitophilus zeamais, Henosepilachna vigintiocto-punctata, Callosobruchus chinensis, Monochamus alternatus and Aulacophora femovalis; Hymenoptera such as Athalia rosae japonensis, and Arge similis; Diptera such as Culex pipiens fatigans, Aedes aegypti, Asphondylia sp., Hylemya platura, Musca domestica vicina, Dacus cucurbitae, and Agromyza oryzae; Aphaniptera such as Pulex irritans, Xenopsylla cheopis and Ctenocephalides canis; Thysanoptera such as Scirtothrips dorsalis, Thrips tabaci, Thrips palmi and Baliothrips biformis; Anoplura such as Pediculus humanus corporis and Pthirus pubis; Psocoptera such as Trogium pulsatorium and Liposcelis bostrychophus; Orthoptera such as Gryllotalpa africana, Locusta migratoria, Oxya yezoensis, Blattella germanica and Periplaneta fuliginosa; and Acarina such as Tetranychus urticae, Panonychus citri, Tetranychus cinnabarinus, Tetranychus kanzawai and Rhizoglyphus echinopus.

The nematodes on which the nematicides of the present invention are effective are Tylenchida such as Heterodera glycines, Meloidogyne javanica, and Pratylenchus penetrans.

The compounds of the present invention can prevent insect pests effectively by contacting them, or control insect pests on leaves by applying the compounds. Thus, the compound is an excellent insecticide,

acaricide or nematicide which has characteristics as a systemic insecticide with scarce phytotoxicity on the host crops.

Now, the present invention will be described by Examples.

EXAMPLE 1 (Extraction)

15 Kg of raw roots of lopseed were washed with water, placed in a large bottle, added with 30ℓ of ethyl ether, and immersed for 2 days at room temperature. After the ether was decanted, further 30ℓ of ether were added and immersion was repeated. 90ℓ of ether extract obtained by three times-immersion, were concentrated to 7ℓ, washed three times by 2ℓ of 5% aqueous solution of sodium hydrogen carbonate, and further washed three times by 2ℓ of water. The obtained ether solution was dehydrated over an anhydrous sodium sulfate, and the ether was removed to obtain 120g of a neutral fraction soluble in ether. This fraction was dissolved in 300 ml of ether, and dropped into 10ℓ of petroleum ether at room temperature under stirring. Thus 105g of a yellow-brown resinous precipitate (crude extract) insoluble in petroleum ether were obtained. The insecticidal activity depended mainly on this precipitate.

EXAMPLE 2 (Extraction)

143 g (weight of raw material) of roots of lopseed were smashed and subjected to extraction by dipping with 1ℓ of methanol for 2 days. A methanol solution obtained by three times repeated extraction was concentrated to obtain 10.5 g of a crude extract, which was applied by liquid to liquid partition with 150 ml of ether and 50 ml of water, twice. The ether layer was concentrated to 3.14 g of crude extract, which was subjected to liquid to liquid partition with 50 ml of acetonitrile and 100 ml of petroleum ether. An acetonitrile layer thus obtained was concentrated to obtain 2.21 g of extract.

EXAMPLE 3 (Extraction)

730 g (weight of raw material) of roots of lopseed were dried by airing in the shade for 2 days, and extracted by dipping with 6ℓ of ethanol for 2 days, repeatedly three times. The ethanol solution obtained was concentrated to about 200 ml, and subjected to partition by addition of 0.5ℓ of ethyl acetate and 1.0ℓ of water. The ethyl acetate layer was washed with water and concentrated to obtain about 10 g of extract. This extract was subjected to liquid to liquid partition with 0.3ℓ of methanol and 0.6ℓ of n-hexane, and the obtained methanol solution was concentrated to obtain 6.6 g of extract.

EXAMPLE 4 (Separation)

10 g of the yellow-brown resinous precipitate described in Example 1, were dissolved in 40 ml of a mixed solvent of isopropylethyl/benzene = 1/5 (v/v), and laid on a column (6.5 cm across, 20 cm high) packed with 400 g of Silicagel 60H (Merck Corp.) by a dry process, and separated chromatographically while varying a mixing ratio of isopropylether to benzene. Every fractions were analyzed according to high-speed liquid chromatography, as well as fractionated while assaying of pesticidal activity.

(Analytical conditions of high-speed liquid chromatography:

Novapak-Silica® (Waters Corp.) 3.8mm x 15cm

ethyl acetate/benzene = 0.5/10 - 1.0/10

linear gradient, 0.5 ml/min UV 300 nm)

As a more active fraction, a part (1.0 g) eluted at Rt = 12 - 18 min according to semiprepatative adsorptive high-speed liquid chromatography (Yamamura-Kagaku, YMC-Pak SH-043 (S-15SIL), 20mm x 25cm) with ethyl acetate/benzene = 1/10 (0.5 ml/min) was separated. This fraction contained the compounds of the formulae (2), (3), (4), (5) and (6) described below, and so by repeated applications of high-speed liquid chromatography to the fraction, about 20 mg of the compounds (2), (3), (4), (5) and (6) were obtained respectively.

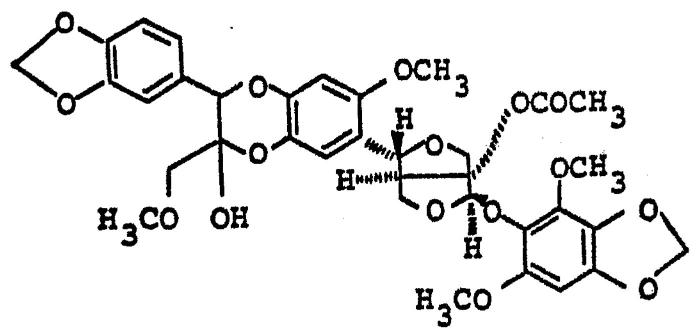

(2)

Elementary analysis: H: 5.16%, C: 59.92%

Calculated as molecular formula ($C_{35}H_{36}O_{16}$): H: 5.09%, C: 58.98%

Properties: a colourless rod crystal, melting at 138 - 140°C.

*PMR spectrum : 2.10 (3H, s), 2.92 (1H, m), 3.41 (3H, s), 3.45 (1H, d, J=10), 3.5 (1H, d, J=10), 3.74 (3H, s), 3.84 (1H, d, J=10), 3.9 (1H, dd), 3.92 (3H, s), 4.6 (1H, dd, J=8,4), 4.67 (1H, d, J=10), 4.74 (3H, s), 4.86 (1H, J=6), 4.95 (1H, s), 5.82 (1H, s), 5.88 (2H, s), 5.96 (2H, s), 6.24 (1H, s), 6.51 (1H, s), 6.81 (1H, d, J=8), 6.96 (1H, dd, J=8,2), 7.12 (1H, d, J=2), 7.18 (1H, s)

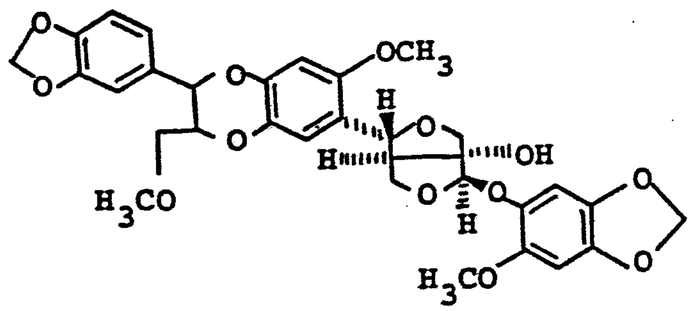

(3)

Elementary analysis: H: 5.13%, C: 61.53%

Calculated as molecular formula $C_{32}H_{32}O_{13}$ : H: 5.16%, C: 61.53%

Properties : a colourless resinous matter

*PMR spectrum : 1.65 (1H, s), 2.64 (1H, m), 3.31 (1H, dd, J=11,4), 3.35 (3H, s), 3.58 (1H, dd, J=11,2), 3.73 (3H, s), 3.74 (1H, d, J=9.6), 3.77 (3H, s), 4.0 (1H, m), 4.09 (1H, dd, J=9,2.5), 4.32 (1H, d, J=9.6), 4.38 (1H, dd, J=9,7.5), 4.95 (1H, d, J=6), 4.99 (1H, d, J=8), 5.19 (1H, s), 5.90 (2H, s), 5.99 (2H, s), 6.49 (1H, s), 6.55 (1H, s), 6.79 (1H, s), 6.81-6.96 (3H, m), 7.31 (1H, s)

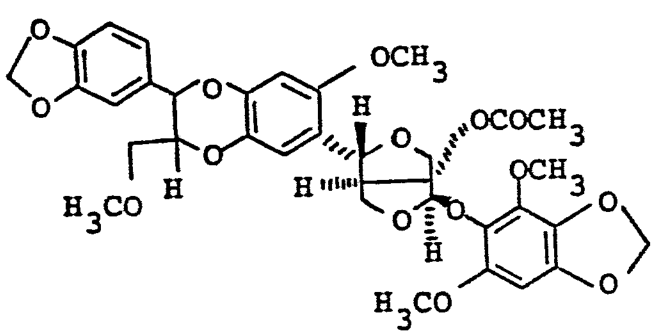

(4)

Elementary analysis: H: 5.19%, C: 60.31%

Molecular formula : $C_{35}H_{36}O_{15}$

Properties: a colourless resinous matter

*PMR spectrum: 2.10 (3H, s), 2.95 (1H, m), 3.28 (1H, dd, J=11,4), 3.35 (3H, s), 3.64 (1H, dd, J=11,2), 3.74 (6H, s), 3.76 (1H, m), 3.85 (1H, d, J=11), 3.95 (3H, s), 3.96 (1H, dd, J=10,2), 4.60 (1H, dd, J=10,6), 4.69 (1H, d, J=11), 4.90 (1H, d, J=6), 5.02 (1H, d, J=8), 5.81 (1H, s), 5.84 (2H, s), 5.96 (2H, s), 6.22 (1H, s), 6.46 (1H, s), 6.76-6.94 (3H, m), 7.17 (1H, s)

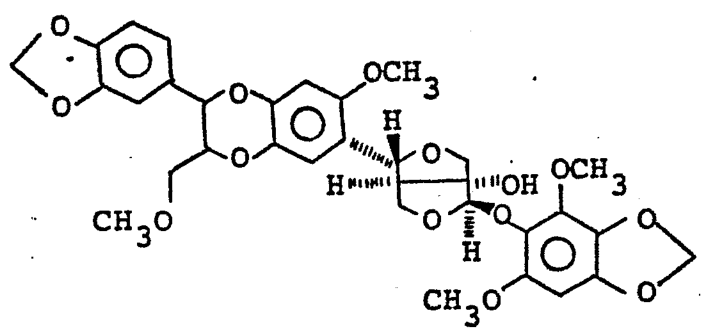

(5)

Elementary analysis: H: 5.37%, C: 60.74%

Calculated as molecular formula $C_{33}H_{34}O_{14}$ : H: 5.23%, C: 60.55%

Properties: a colourless crystal, melting at 158-159°C.

*PMR spectrum: 1.6 (1H, s, OH), 2.64 (1H, m), 3.30 (1H, dd, J=10.7, 3.9), 3.35 (3H, s),

3.59 (1H, dd, J=10.7, 2.0), 3.73 (3H, s), 3.74 (2H, d, J=9.3), 3.79 (3H, s), 4.0 (1H, m), 4.02 (3H, s), 4.05 (2H, dd, J=8.8, 2.0), 4.30 (2H, d, J=9.3), 4.57 (2H, dd, J=8.8, 6.8), 4.94 (1H, d, J=5.9), 4.99 (1H, d, J=7.8), 5.24 (1H, s), 5.88 (2H, s), 6.00 (2H, s), 6.29 (1H, s), 6.49 (1H, s), 6.83-6.93 (3H, m), 7.31 (1H, s)

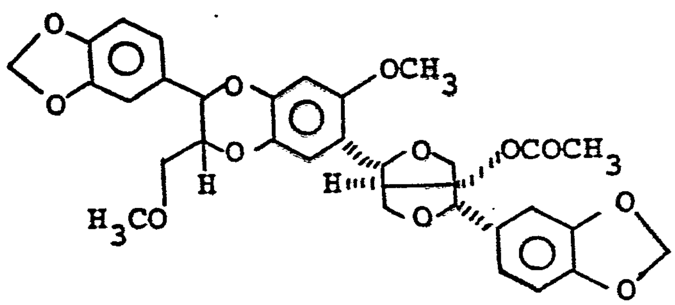

(6)

Elementary analysis: H: 5.22%, C: 63.84%

Calculated as molecular formula $C_{33}H_{32}O_{12}$ : H: 5.20%, C: 63.86%

Properties: a colourless resinous matter

*PMR spectrum: 1.72 (3H, s), 3.14 (1H, m), 3.26 (1H, dd, J=11, 4), 3.32 (3H, s), 3.58 (1H, dd, J=11, 2), 3.76 (3H, s), 3.88 (1H, dd, J=10, 4), 3.96 (1H, m), 4.28 (1H, d, J=10), 4.39 (1H, d, J=10), 4.45 (1H, dd, J=10, 7), 4.92 (1H, s), 4.96 (1H,

d, J=8), 4.98 (1H, d, J=5), 5.92 (2H, s), 5.96 (2H, s), 6.48 (1H, s), 6.65-6.92 (6H, m), 7.10 (1H, s)

EXAMPLE 5 (Separation)

10 g of the yellow-brown resinous precipitate described in Example 1, were dissolved in 40 ml of a mixed solvent of ethyl acetate/dichloromethane = 1/7.5 (v/v), laid on a column (4 cm across, 20 cm high) packed with 400 g of Silicagel 60 (Merck Corp.) (230 to 400 mesh) by a wet process, and separated by chromatography, while varying a mixing ratio of ethyl acetate to dichloromethane.

A fraction of high activity was concentrated and dissolved in acetonitrile, then according to high-speed partition liquid chromatography (Waters Corp., Novapak® $C_{18}$ 39mm x 15cm) with $MeCN/H_2O$ = 1/1 (1.0 ml/min, UV 300 nm), a part eluted at Rt = 10 to 15 min was separated. This fraction contained the compounds (2), (3), (4), (5) and (6) and was further applied to semi-preparative adsorptive high-speed liquid chromatography to obtain about 20 mg of each compounds of the formulae (2), (3), (4), (5) and (6).

In addition, data of *PMR of Examples 2 - 6 show a chemical shift (ppm) observed in $CDCl_3$ chloroform by JEOL, FX-100.

Formulation Example

Formulation Example 1. Emulsifiable concentration

2 parts of a compound of the present invention was dissolved in 18 parts of isophorone and 65 parts of a mixed solution of xylene/methylnaphthalene (5:5). 15 parts of a mixture of an alkylphenol-ethylene oxide condensate and calcium alkylbenzenesulfonate (8 : 2) were mixed with the obtained solution to prepare an emulsifiable concentration. This emulsifiable concentration may be used as a spreading agent by diluting water.

Formulation Example 2. Wettable powder

2 parts of a compound of the present invention were mixed with 35 parts of kaolin, 48 parts of clay and 7.5 parts of diatomaceous earth. 7.5 parts of a mixture of sodium lauryl sulfate and β-naphthalenesulfonic acid/formalin condensate in ratio of 1 : 1 were added to the obtained mixture. The resulting mixture was finely ground to prepare a wettable powder. This wettable powder may be used as a spreading agent by diluting with water.

Formulation Example 3. Dust

0.5 part of a compound of the present invention was mixed with 99.2 parts of a mixture of talc and calcium carbonate (1 : 1) and 0.3 part of isopropyl acid phosphate. The resulting mixture was homogeneously dispersed, blended

and further ground to prepare a dust. This dust may be used as a spreading agent as such.

Formulation Example 4. Granule

0.5 part of a compound of the present invention was mixed with 48 parts of finely powdered bentonite, 49.5 parts of talc and 2 parts of sodium ligninsulfonate, followed by the addition of water. The resulting mixture was kneaded until it became homogeneous. The mixture was granulated by passing it through an injection molding machine and adjusted to a granular size of 0.6 to 1 mm by passing the granule thus molded through a spherizer and a drying screen elassifier. The obtained granule may be directly spreaded on the surface of paddy fields and uplands as such.

Formulation Example 5. Oil

A mixture of 0.1 part of a compound of the present invention and 0.5 part of piperonyl butoxide was mixed with 10 parts of isophorone, and further dissolved in such an amount of illuminating kerosine as to give the total volume of 100 parts to prepare an oil. This oil may be used as such.

Formulation Example 6. Aerosol

0.4 part of a compound of the present invention, 20 parts of piperonyl butoxide, 4 parts of isophorone, 6 parts of xylene and 3.6 parts of deodorized kerosine were

mixed and dissolved. After filling the mixture into an aerosol container, a valve was fitted. 86 parts of Freon were introduced into the container through the valve under pressure to obtain an aerosol.

Test Example 1: Contact Toxicity to Housefly

1 μℓ of active ingredient solution diluted with acetone was treated topically at a dorsal thorax of 3 to 5 days old female housefly using a micro syringe. 10 treated flies per a dosage were replaced into a plastic cup together with cotton containing 5% sucrose solution. Mortalities of the flies were checked 24 hours after treatment. Two replications were used for a dosage and the test was conducted under 23 ± 1°C.

Test Result: Contact Toxicity to Housefly

| Active Ingredient | Mortality at indicated dosage (μg/fly) | | | | | |
|---|---|---|---|---|---|---|
| | with Pb* | | | | without Pb* | |
| | 3 | 0.3 | 0.006 | 0.0012 | 3 | 0.3 |
| Compound (3) | 100 | 100 | 100 | 60 | 100 | 95 |
| Compound (2) | 100 | 100 | 80 | 30 | 0 | 0 |
| Compound (4) | 100 | 90 | 50 | 10 | 20 | 0 |
| Compound (2), (3), (4), (5) and (6) | 100 | 95 | 35 | 15 | 40 | 0 |

*Pb: piperonyl butoxide

Test Example 2: Oral Toxicity to Common Cut Worm, Spodoptera litura

1 μℓ of Acetone solution containing the active ingredients were treated on 8 x 8 $mm^2$ of cabbage leaves and fed to 5th instar larvae (200 to 300 mg in body weight) of Common Cut Worm, then after one hour artificial diets (Insecta LF® produced by Nippon Nohsan Kogyo KK) were fed to the larvae. One day after feeding the treated leaves, mortalities of larvae were checked.

Test Result 2: Oral toxicity to Common Cut Worm

| | Mortalities of larvae at indicated dosages (μg/larva) | | | | | |
|---|---|---|---|---|---|---|
| | 100 | 33 | 10 | 3.3 | 1.0 | 0.33 |
| Compound (3) | 100 | 100 | 100 | 100 | 50 | 22 |
| Compound (2) | 100 | 100 | 100 | 30 | 0 | 0 |
| Compound (5) | 100 | 100 | 100 | 100 | 60 | 15 |
| Acephate | 100 | 100 | 100 | 10 | 0 | 0 |

## CLAIMS

1. A sesquilignan derivative of the formula:

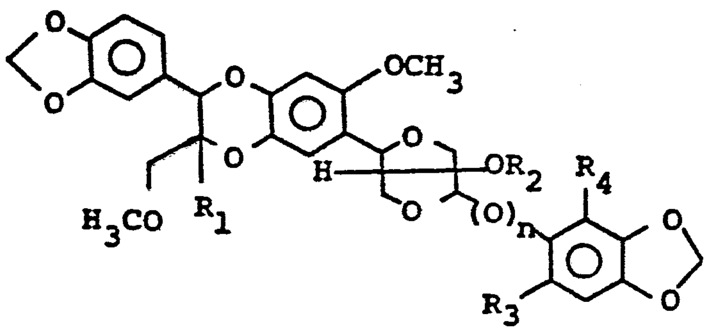

wherein $R_1$ is hydrogen or hydroxy, $R_2$ is hydrogen or acetyl, $R_3$ is hydrogen or methoxy, $R_4$ is hydrogen or methoxy and n is 0 or 1, with the proviso that when $R_1$ and $R_2$ are each hydrogen, $R_3$ is methoxy and $R_4$ is hydrogen.

2. A derivative according to claim 1 wherein $R_1$ is hydroxy, $R_2$ is acetyl, $R_3$ and $R_4$ are each methoxy and n is 1.

3. A derivative according to claim 1 wherein $R_1$, $R_2$ and $R_4$ are each hydrogen, $R_3$ is methoxy and n is 1.

4. A derivative according to claim 1 wherein $R_1$ is hydrogen, $R_2$ is acetyl, $R_3$ and $R_4$ are each methoxy and n is 1.

5. A derivative according to claim 1 wherein $R_1$, $R_3$ and $R_4$ are each hydrogen $R_2$ is acetyl and n is 0.

6. A pesticidal composition containing, as active ingredient, a mixture of sesquilignan derivatives of the formula:

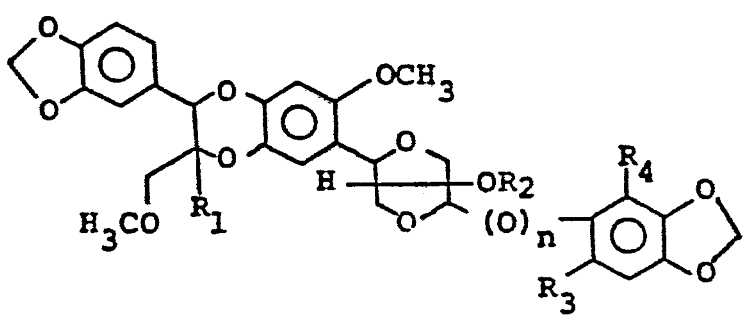

(1)

wherein $R_1$ is hydrogen or hydroxy, $R_2$ is hydrogen or acetyl, $R_3$ is hydrogen or methoxy, $R_4$ is hydrogen or methoxy and n 0 or 1.

7. A method for obtaining a crude extract containing a mixture of sesquilignan derivatives as defined in claim 6, which method comprises

(1) extracting lopseed (Phryma leptoslachya L.) with an organic solvent and removing solvent from the extract solution to provide a concentrate,

(2) removing materials which are soluble in an acidic, alkaline or neutral aqueous solution by partition of the concentrate from (1) between an organic solvent and said aqueous solution and concentrating the organic solution by removing said organic solvent, and

(3) removing nonpolar-solvent-soluble materials

(a) by partition of the concentrate from (2) between a nonpolar solvent and a polar solvent and concentrating the polar-solvent solution to obtain a crude extract, or

(b) by addition of the organic solution from (2) to a large volume of a non-polar solvent with stirring and decanting the non-polar solvent to obtain a crude extract.

8. A method according to claim 7 wherein the crude extract from step (3) is chromatographed to separate the individual sesquilignan derivatives of the mixture thereof in the crude extract.

9. Use of a sesquilignan derivative as claimed in any one of claims 1 to 5 or a composition as claimed in claim 6 as a pesticide.